(19) Europäisches Patentamt

European Patent Office

Office européen des brevets

(11) **EP 3 477 288 B1**

(12) **EUROPEAN PATENT SPECIFICATION**

(45) Date of publication and mention
of the grant of the patent:
**22.06.2022 Bulletin 2022/25**

(21) Application number: **17198676.3**

(22) Date of filing: **26.10.2017**

(51) International Patent Classification (IPC):
**G01N 21/898** (2006.01)    **G01N 3/56** (2006.01)
**G01N 33/36** (2006.01)

(52) Cooperative Patent Classification (CPC):
**G01N 21/8983; G01N 33/367;** G01N 3/56

(54) **A METHOD OF MEASURING STAIN REMOVAL**

VERFAHREN ZUR MESSUNG EINER FLECKENENTFERNUNG

PROCÉDÉ DE MESURE D'ÉLIMINATION DE TACHES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.05.2019 Bulletin 2019/18**

(73) Proprietor: **The Procter & Gamble Company
Cincinnati, OH 45202 (US)**

(72) Inventor: **AMADOR ZAMARRENO, Carlos
Newcastle upon Tyne, NE12 9TS (GB)**

(74) Representative: **P&G Patent Belgium UK
N.V. Procter & Gamble Services Company S.A.
Temselaan 100
1853 Strombeek-Bever (BE)**

(56) References cited:
**CN-A- 105 628 563    DE-A1-102006 029 485
GB-A- 653 657    US-A- 2 561 133**

**Description**

FIELD OF THE INVENTION

The present invention relates to a method of measuring stain removal.

BACKGROUND OF THE INVENTION

Equipment for measuring kinetic friction is presented by Hailing in the book: Introduction to tribology on page 61 (Halling, 1976). This device measures dynamic friction coefficients between materials. In the present invention, there are many novel features to develop this kinetic friction measurement equipment into equipment that is suitable for use in a method for measuring stain removal. For measuring stain removal from fabrics the following features were developed: a heated bath with chemistry solution, addition of independently controlled abrasive, compressive and stretching forces on the stained fabric, a camera system, which works in combination with an online image analysis software and a visual cue system on the stain fabric that allows to measure the stain removal of many moving stains simultaneously over time.

The benefits of the present invention include: tight control on the mechanical action (abrasion, compression) application onto the stains which reduces variability between runs; recording of actual forces and motor torque, wet friction coefficient (which can change with load and chemistry), kinetics of cleaning for many stains simultaneously within the same run; reduced man power since there is no need to measure colour of stains before and after wash as this is automatically done by the apparatus; efficient development of stain removal and whiteness models.

Since stain removal data is obtained over time, it is possible to carry out dynamic changes in the inputs (forces, pH, Temperature, water hardness, chemistry concentrations, etc) within the same experimental run. These changes will yield additional features in the stain removal or whiteness curve, allowing for fitting of coefficients that correlate these inputs with the stain removal index (SRI) or change in fabric colour (dE) outputs simultaneously. In this way, for example, it is possible to model the dependency of a new chemical technology with all the varying inputs conducting a very small number of experiments.

US 2 561 133 relates to wear testing apparatus and is concerned primarily with a wear tester designed for use on paper making felts and comparable fabrics.

CN 105 628 563 relates to a rapid measuring method of detergency of washing agents on fabric, and to a measuring device thereof.

GB 653 657 relates to an apparatus for testing the fastness properties, particularly fastness to washing and milling, of dyeings on textile material or yarn, under wet conditions.

DE 10 2006 029485 relates to a device for determining the efficiency of a washing process.

SUMMARY OF THE INVENTION

The present invention provides a method of measuring stain removal of multiple stains from stained fabric (1), wherein the stained fabric (1) comprises one or more, preferably two or more different types of stains, according to claim 1.

BRIEF DESCRIPTION OF THE DRAWINGS

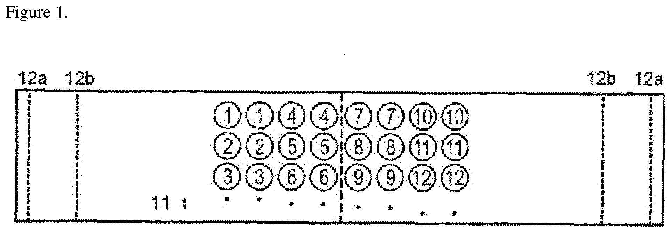

Figure 1 - Stained fabric (1) showing stains, visual cues (11) and location of stitches (12a & 12b).

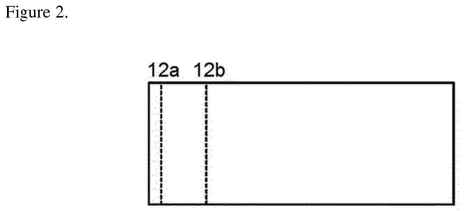

Figure 2 - Stitched stained fabric (1) ready for placement on rotating drum (4).

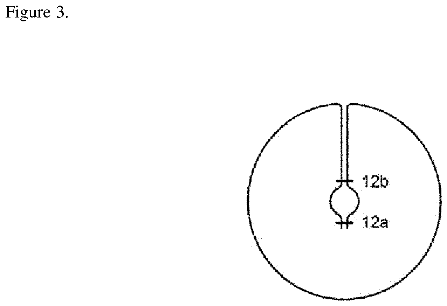

Figure 3 - Stitched stained fabric (1) in a typical configuration when placed on the rotating drum (4).

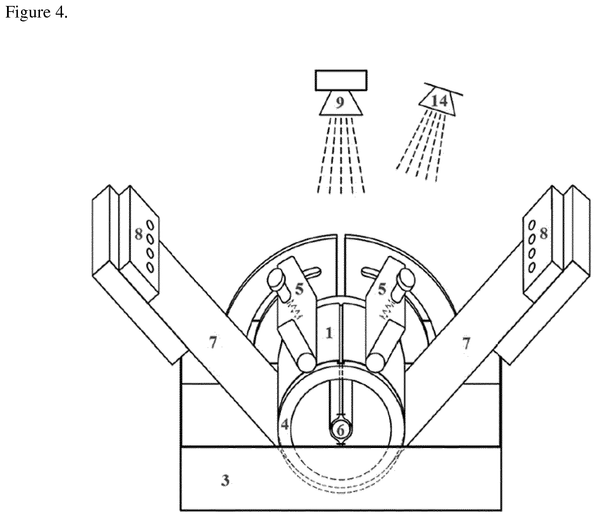

Figure 4 - The apparatus.
Figure 5 - Rotating drum (4).

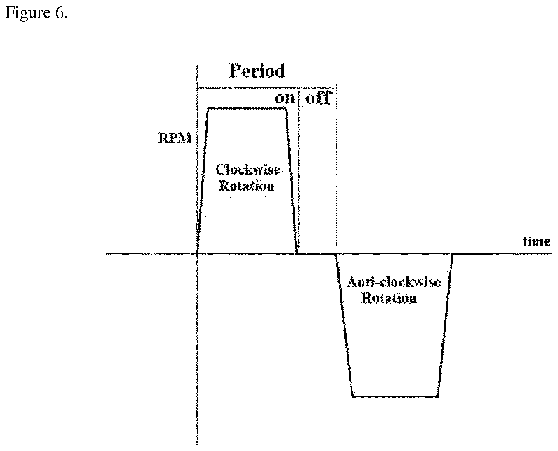

Figure 6 - Plot of rotational speed versus time for rotating drum (4).

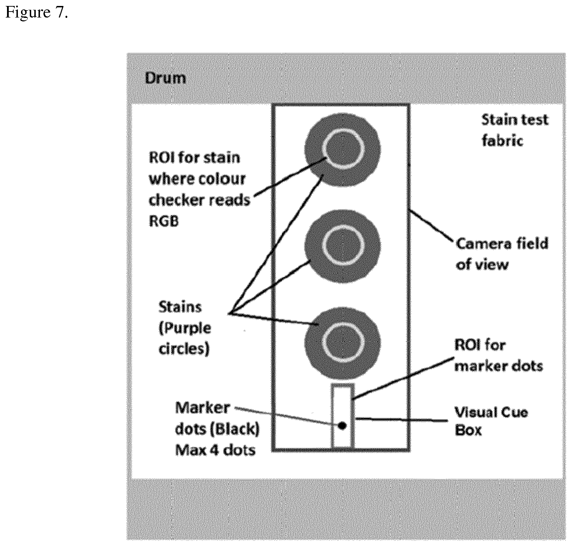

Figure 7 - Image analysis detection means
Figure 8 - Calculation of stain removal
Figure 9. Example of SRI time dependent curves obtained with apparatus for a Gravy stain at two different normal loads (30 and 120 N).
Figure 10. Standard exponential response with time.
Figure 11. SRI curve showing multiple time dependent features due to changes in inputs during the wash.
Figure 12. Experiment 1 of bump test for example 1. Model curve vs. experimental curve.
Figure 13. Experiment 2 of bump test for example 1. Model curve vs. experimental curve.
Figure 14. Experiment 3 of bump test for example 1. Model curve vs. experimental curve.

Figure 15. Experiment 4 of bump test for example 1. Model curve vs. experimental curve.
Figure 16. SRI time dependent curve obtained with apparatus as pH is varied through the wash.

DETAILED DESCRIPTION OF THE INVENTION

Method of measuring stain removal: The present invention provides a method of measuring stain removal of multiple stains from stained fabric (1), wherein the stained fabric (1) comprises one or more, preferably two or more different types of stains, wherein the stained fabric (1) is placed in an apparatus (2), wherein the apparatus (2) comprises: a wash bath (3); rotating drum (4); compressive roller (5); means of applying tension to the stained fabric (6); unstained static fabric (7); means of applying tension to the static fabric (8), which will translate into abrasion force onto the stained fabric; image capture means (9); and image analysis means (10), wherein the stained fabric (1) is secured under tension on the rotating drum (4), wherein during each rotation, each type of stain on the stained fabric (1) is repeatedly contacted to the wash bath (3) at least once per rotation, wherein during each rotation, each type of stain on the stained fabric (1) is contacted to the compressive roller (5) which applies a compressive force to the stain, characterized in that the image capture means (9) is positioned such that the image capture means (9) measures in real time the light absorbance of each type of stain on the stained fabric (1) at least once during each rotation, wherein the stained fabric (1) comprises a visual cue (11), wherein the image capture means (9) can identify the visual cue (11), and the image analysis means (10) can associate the measurement reading for a type of stain with the visual cue for that type of stain , and distinguish between different types of stain on the stained fabric and in this manner read multiple different types of stains or sets on the same stained fabric.

The stains typically rest on the stained fabric that is wrapped around the rotaing drum and thus the in-wash image analysis of moving stains via the specific image capture and analysis means is in real time.

Stained fabric (1): The stained fabric (1) comprises one or more, preferably two or more different types of stains. The stained fabric (1) comprises a visual cue (11).

Typically, the stained fabric is prepared by applying stains directly onto the fabric (direct application of stains), or by an indirect method, such as stitching swatches of certain EMPA, CFT and/or AISE stains onto the stained fabric.

The stained fabric typically has an unstained portion of the fabric, or "space" on the fabric for the image capture means to measure the background ("white" or "unstained" fabric portion) of the stained fabric over time. This can be used as a reference when determining the stain removal from the stained fabric or to study whiteness maintanence.

The stained fabric is typically wrapped around the rotaing drum and placed under stretching tension via a mechanical mechanism to avoid wrinkling/folding of the fabric during the application of abrasive force via the unstained static fabric.

The stained fabric is typically any fabric type, such as cotton, polycotton, polyester, etc. Preferably the stained fabric is of a fabric structure that is not very stretchable. The stains can be directly applied onto the fabric or ready-made stains can be stitched onto the fabric as well. Depending on the size of the stains, a different number of them can be used. A standard set up is shown in the below figure with 24 stains and 2 internal repeats of each stain type.

Typically, height of the visual cue is used by the image capture means together with the image analysis means to associate that column with a specific set of stain types. Typically, two visual cues are used to read the colour of the fabric background during the wash for use in the calculation of stain removal.

Typically, once the stained fabric is prepared with stains and visual cues, it is sewed along paths 12a and 12b to create a fabric loop that fits around the rotating drum.

Apparatus (2): The apparatus (2) comprises: a wash bath (3); rotating drum (4); compressive roller (5); means of applying tension to the stained fabric (6); unstained static fabric (7); means of applying tension to the static fabric (8); image capture means (9); and image analysis means (10).

Wash bath (3): The wash bath is typically fabricated in stainless steel 316 and can hold a variable volume of water, such as between from 2 and 20 litres. Fabrics, such as ballast fabric, can be added to the solution in the wash bath in order to modify the ratio of water to fabric, and/or chemistry to fabric.

The wash bath can typically move up and down via a hydraulic arm so that the portion of the rotating drum that is immersed in solution can be changed. A heating coil fed by heated water may be used to maintain or change the temperature of the wash bath without putting the wash solution in contact with very high temperatures that could affect some of the detergent actives. The rotation of the rotating drum typically causes agitation of the wash bath although an additional stirrer can be placed into the wash bath to improve mixing of the wash solution.

A variety of different chemistries can be introduced into the wash bath, for example simple individual chemistries such as a surfactant, to combination of chemistries such as a surfactant and polymer, to complex chemical combinations such as a fully formulated laundry detergent composition (comprising surfactants, polymers, builders, enzymes, bleach, perfume, fillers, dyes, etc) dissolved in water.

Rotating drum (4): The rotating drum typically features a smooth surface for the stained fabric to accommodate well. The rotating drum typically has a slit (4a), typically broad enough to allow the stained fabric region 12a-12b to fit around the internal shaft of the rotating drum so that the stained fabric can be stretched to a given tension by moving the shaft down via a screwed mechanism.

The rotating drum is typically controlled by a motor and a drive software (e.g. Ultraware) that allows to change the rotational speed, preferably from 0 to 60 rpm, the direction of rotation, and the on/off times within each period.

Compressive roller (5): The apparatus can comprise more than one compressive roller, e.g. at least two, or at least three or more compressive rollers.

The compressive roller typically applies only a compressive force and not an abrasive force to the stained fabric because the compressive roller rotates so that there is no dragging, or at least no significant dragging, on the surface of the stained fabric.

The compressive rollers are typically spring loaded and a different compressive force can be set by the use of a screw micrometer. As the rotating drum rotates, the compressive rollers typically provide a compression force on the stained fabric, typically without causing abrasion, because they rotate. The compression force can be calculated as the equation below where the displacement is calculated from the screw thread length and the screw number of turns.

$$F = 2*k_{Spring}*\text{displacement}$$

The compressive rollers typically have two functions. Firstly, they remove excess of wash solution from the stained fabric before the stains get under the field of view of the image capture means so that a clear image without reflection can be taken by the image capture means. When rotation in both directions takes place, preferably two compressive rollers are used, with one at either side of the image capture means' field of view. Secondly, adjust the compressive force on the stained fabric, which impacts stain removal in an independent way from the abrasive force.

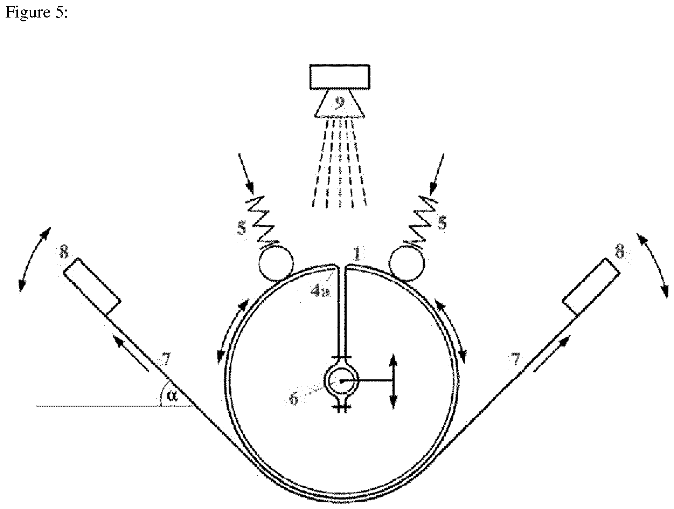

Means of applying tension to the stained fabric (6): Means of applying tension to the stained fabric includes: moveable shaft within the drum, springs, piston arms. Preferably, the stained fabric (1) is wrapped around the drum and it goes inside the drum through a drum slit (4a). Then, preferably the stained fabric goes around the drum shaft and back out of the drum through the same drum slit (4a). Preferably, the shaft is mounted onto a moveable plate at the back of the drum that can be adjusted up and down via a screw mechanism in order to set up a different stretching force on the fabric (see Figure 5).

Unstained static fabric (7): The use of unstained static fabric, typically that stretches very little, is preferable so that tension does not need to be readjusted over time. A different material with different friction coefficient can be stitched on the unstained static fabric in order to increase the abrasion of the stained fabric. Additionally, a completely inelastic material can be used instead, to which a piece of fabric is attached at the point of contact with the stained fabric.

Means of applying tension to the static fabric (8): The means of applying tension to the static fabric is typically one or more tension arms, typically two or more tension arms. The tension arms are typically operated via screw micrometers for precise setting of the loads. Each tension arm is typically connected to a load cell so that the applied force in each tension (such as from 0 to 150 N) can be measured and recorded. Typically, the tension arms can also be moved up and down in a circular fashion so that the contact area between the unstained fabric (7) and the stained fabric (1) can be changed. This also changes the amount of perpendicular compressive load between the two fabrics.

Image capture means (9): The image capture means (9) is positioned such that the image capture means (9) measures in real time the light absorbance of each type of stain on the stained fabric (1) at least once during each rotation.

Typically, the image capture means is a camera, preferably a colour camera. A colour camera is typically needed in order to record RGB colour components of the stains. Typically, the colour camera needs to have manual control of the exposure (aperture) in order to get the right light depending on the light source, if any, being used. Typically, the colour camera also needs to have a high enough frame rate which can be calculated from the maximum desirable rotating speed of the roating drum ($RPM_{max}$), the drum radius ($r_{drum}$) and the distance where the stain visual cue is active ($l_{visualcue}$) for each stain column, which is defined within the image analysis software.

$$\text{Frame rate} = 2*\pi*r_{drum}*(RPM_{max}/60)/l_{visual\ cue}$$

For example, for a 7.5 cm radius rotating drum, a maximum rotational speed of 60 rpm and a lvisual cue = 5 mm, the minimum frame rate required is

$$\text{Frame rate} = 2*\pi*0.075*(60/60)/0.005 = 95\ \text{frames/s}$$

The image capture means (9) can identify the visual cue (11), and the image analysis means (10) can associate the measurement reading for a type of stain with the visual cue, and distinguish between different types of stain on the stained fabric and in this manner read multiple different types of stains or sets on the same stained fabric.

Image analysis means (10): The image analysis means (10) can associate the measurement reading for a type of stain with the visual cue for that type of stain, and distinguish between different types of stain on the stained fabric.

Typically, the image analysis means is an image analysis software application. A suitable image analysis software application has been customised for this application which has the following features:

(a) only visual cues with a specific trained colour, size and vertical height are detected;
(b) camera only takes pictures when each visual cue, which is aligned with the centre of the stains, is within a visual cue box, typically from 0.5 to 1.0 cm width;
(c) each picture records the R G B components of as many regions of interests as stains there are in that specific column;
(d) in order to avoid having the regions of interest outside the stains, the diameter of the stain Regions of Interest (ROI) has to be < (diameter of the stain - visual cue box width).

Data Recording: Typically, the following data is recorded in a computer via a data acquisition card: rotational speed of the drum, left tension arm force, right tension arm force and motor torque. The wet friction coefficient during the wash can be estimated from the dynamic loads recorded on the tension arms load cells.

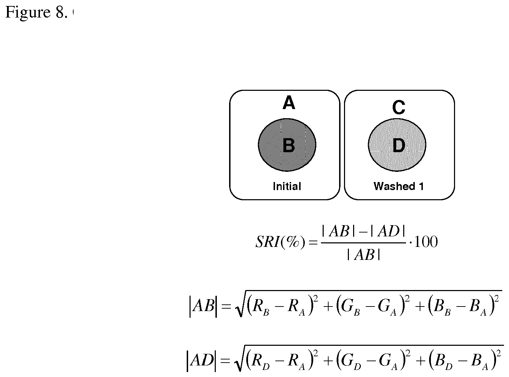

Calculation of stain Removal: Typically, at any given time, we can calculate the percentage of stain colour removed via the equation below, using the R G B components of the stains captured by the camera. R G B components can also be converted into L A B components for a given light source if colour distances in the L A B space are to be used. An example using the RGB components is shown in figure 8.

SRI values calculated in wet conditions with RGB components are then calibrated SRI values of the same fabrics calculated after drying using LAB components.

Visual cue (11): Typically, one visual cue (11) per column of stains is needed. There are different ways to define a set of distinguishable cues, such as a different number of dots per column. An approach that works well for a large number of stain columns is to use a single dot as the visual cue where its height on the fabric changes per column of stains. In order to avoid mistaking other objects (e.g. stains or drum slit (4a)) with the visual cues, the image analysis means (10), typically sets boundaries on the features of the visual cues: e.g. a small range of colour from a trained colour, a maximum and a minimum size for the visual cue to be detectable, and a visual cue box region where the image analysis means (10) searches for visual cues.

Light Source (14): A light source may be used. Different types of light sources may be used. Since the frame rate of the camera is not very high, the aperture does not need to be very 1. Typically the main purpose of the light source is to provide considerably much more light than the ambient light so that any ambient light fluctuations will not impact the results. A LED line light with integrated intensity control is adequate for this application providing bright and uniform flooding light to the stained fabric.

EXAMPLE

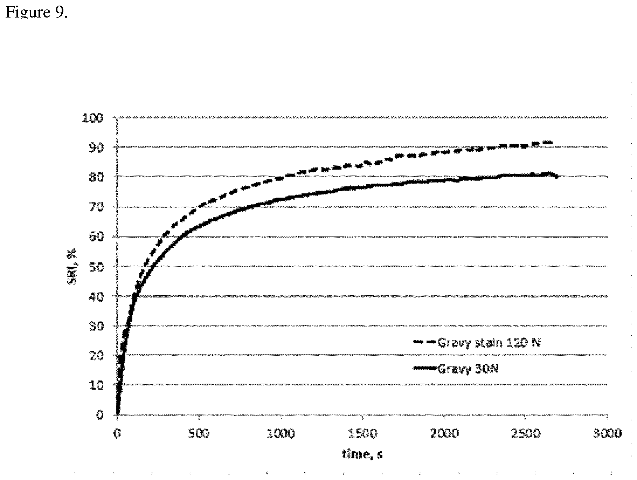

Data Obtained from Device: Figure 9 shows Stain Removal Index for a Gravy stain run under two different abrasion forces (normal loads between the stained and static fabric of 30 N and 120 N respectively).

Development of Performance (Stain Removal and Whiteness) Models via Dynamic Change of Inputs: The example below describes the experimental procedure enabled by this new device which allows to fit mathematical models that correlate wash inputs and chemical inputs to Stain Removal or Whiteness performance. Many different mathematical models can be used to fit the experimentally obtained curves. If we take a simple case where the Stain Removal curve follows an exponential curve, this can be defined by 2 parameters:

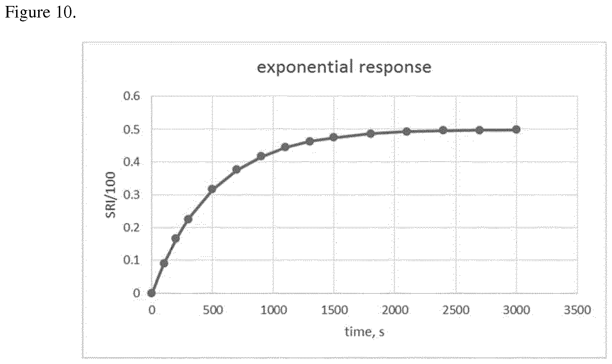

$$SRI = SRI_{Max} \cdot \left(1 - \exp(-k \cdot t)\right) \text{ (for SRIMax = 0.5 and k = 0.002, figure 10 is obtained)}$$

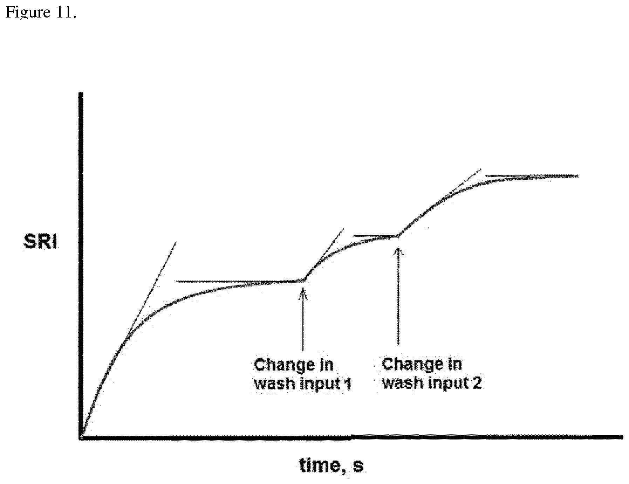

The more features on the cleaning curve, the more coefficients that can be fitted. For example figure 11, would allow to fit at least 6 coefficients, 2 per each exponential section.

This equation can also be written in differential form as the equation below, so that k (rate constant) and $SRI_{max}$ (maximum value of stain removal) will dynamically change if some of the wash inputs (force, temperature, pH, chemistry concentrations, etc) are changed during the wash:

$$\frac{dSRI}{dt} = k \cdot \left(SRI_{max} - SRI\right)$$

$$k, SRI_{Max} = f(force, T, pH, hardness, chemistry)$$

Example 1

For example, in order to model the impact of a chemical technology (e.g. protease enzyme) on stain removal as a function of temperature, hardness, abrasion force and time, only 4 experiments are needed, where the inputs are varied over time via step or ramp changes:
• Experiments 1 & 2 map the interactions of the base detergent chassis with temperature, hardness, mechanical action and time so that its effects can be discounted from the effect of the chemistry that will be studied.

| | **Experiment 1** | | | |
|---|---|---|---|---|
| Time (mins) | Force, N | Temp, $^0$C | pH | hardness, gpg |
| 0-20 | 30 | 20 | 8 | 4 |
| 20-40 | 30 | 20 | 8 | 30 |
| 40-60 | 90 | 20 | 8 | 30 |
| 60-90 | 30 | 40 | 8 | 30 |
| 90-110 | 90 | 40 | 8 | 30 |
| | **Experiment 2** | | | |
| Time (mins) | Force, N | Temp, $^0$C | pH | hardness, gpg |
| 0-20 | 30 | 20 | 8 | 4 |
| 20-40 | 90 | 20 | 8 | 4 |
| 40-70 | 30 | 40 | 8 | 4 |
| 70-90 | 90 | 40 | 8 | 4 |

• Experiments 3 & 4 map the benefits of the chemical technology under study (e.g. protease enzyme) on top of the base detergent chassis and its interactions with Temperature, hardness, mechanical action and time

Experiment 3 - Chemical Technology Level study

| Time (min) | pH | Temp (Celsius) | Hardness (gpg) | Force (N) | Cumulative Protease Enzyme Concentration |
|---|---|---|---|---|---|
| 0-20 | 8 | 20 | 4 | 30 | 0 |
| 20-40 | 8 | 20 | 4 | 30 | 0.27 |
| 40-60 | 8 | 20 | 4 | 30 | 1.2 |
| 60-80 | 8 | 20 | 4 | 30 | 2.4 |
| 80-100 | 8 | 20 | 4 | 90 | 2.4 |

Experiment 4 - Chemical Technology Condition Study

| Time (min) | pH | Temp (Celsius) | Hardness (gpg) | Force (N) | Cumulative Protease Enzyme Concentration |
|---|---|---|---|---|---|
| 0-20 | 8 | 20 | 4 | 30 | 0 |
| 20-40 | 8 | 20 | 30 | 30 | 1.2 |
| 40-60 | 8 | 20 | 30 | 90 | 1.2 |

(continued)

| Time (min) | pH | Temp (Celsius) | Hardness (gpg) | Force (N) | Cumulative Protease Enzyme Concentration |
|---|---|---|---|---|---|
| 60-80 | 8 | 40 | 30 | 30 | 1.2 |
| 80-90 | 8 | 40 | 30 | 30 | 1.2 |
| 90-110 | 8 | 40 | 30 | 90 | 22 |

The first 20 minutes of all 4 experiments use the same wash condition so that any noise from one external run to another can be discounted for.

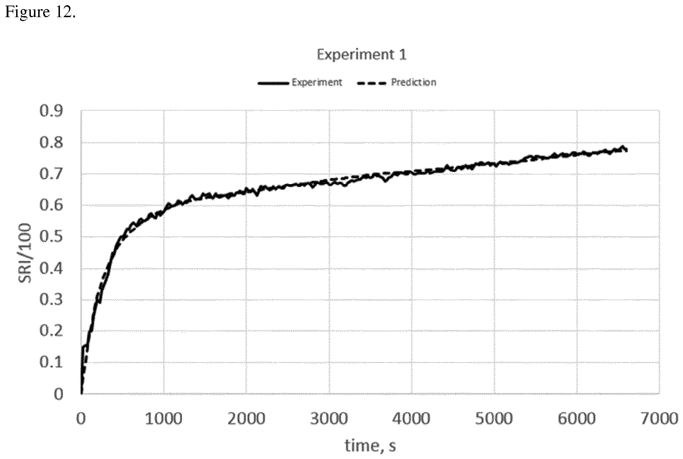

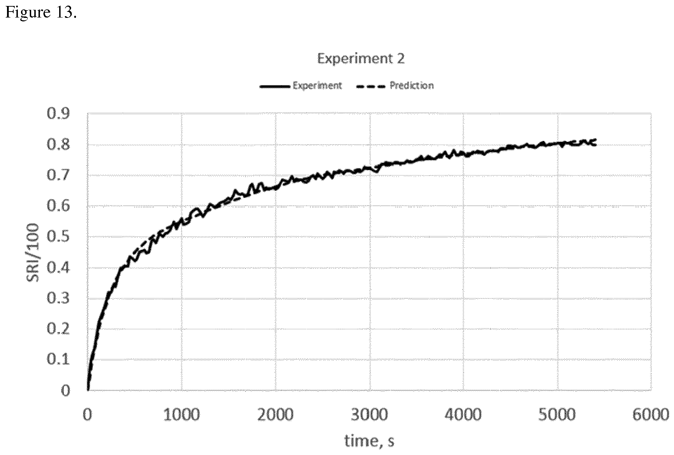

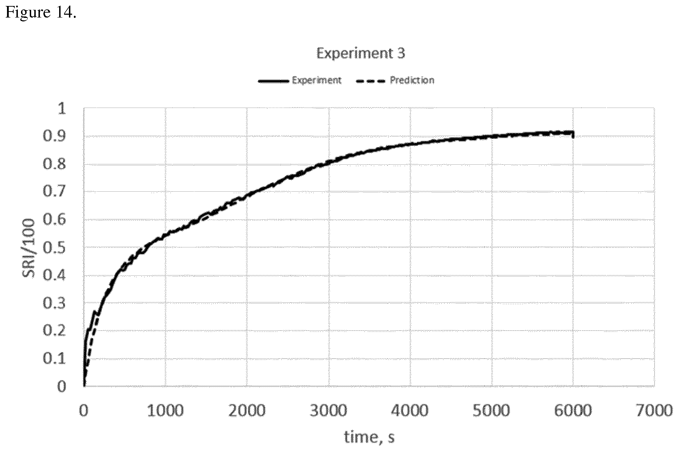

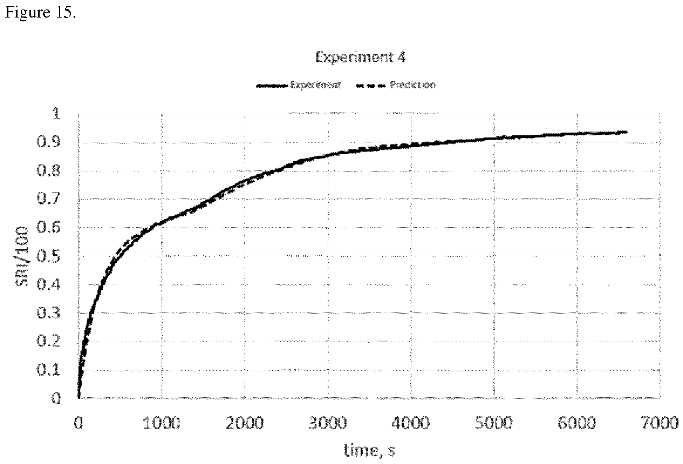

These results for these 4 experiments (figures 12, 13, 14 and 15) are shown (experimental vs. fitted model). All four experimental curves are fitted via a differential mathematical model where the injections of enzyme and the changes in hardness and temperature over time can be simulated. During this process, a number of model coefficients are fitted using a parameter estimation algorithm available in some commercial software packages with Ordinary Differential Equation solvers.

This approach provides additional benefits vs. traditional approaches:

- Efficiency in number of experiments required for modelling the cleaning kinetics of new technologies: A reduced number of experiments is needed because of a) having many points in time for the Stain removal Index; b) being able to record changes in the stain removal during the same experiment by changing the same wash inputs over time. In order to be able to map the impact of the aforementioned input variables with a similar level of accuracy (temperature, hardness, force, time and chemistry concentration) in a standard washing machine experiment where each experiment only provides a value of stain removal, at least 72 experiments may be needed.

- Higher accuracy on the benefit of a specific technology or impact of a specific wash condition is achieved since the baseline on stain removal comes from the same experiment (same stain, eliminating stain to stain variability).

- By having the same condition during the first minutes of the experiment across all the external runs (4 in the example above), it is possible to correct for external variability from run to run. This variability can come from variability in the stain preparation procedure.

<u>Example 2</u>

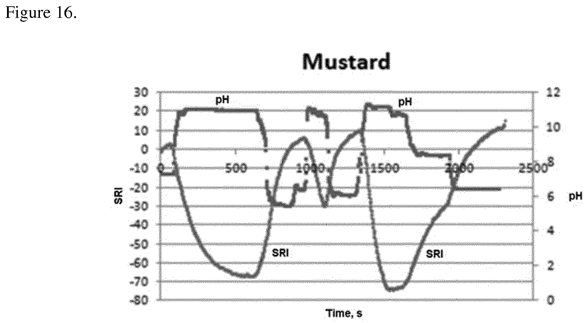

Studying the impact of pH on stain darkening. Figure 16 shows the SRI for a Mustard stain as a function of the actual pH, which is being dynamically change over time between the values of 5.5 and 11. A single experiment is able to provide kinetics of stain darkening due to pH variation during the wash, magnitude of the darkening and features about the reversibility of the process.

In order to generate the SRI curve in a standard washing machine experiment, it would be required to at least run 30-40 experiments in order to generate a similar level of understanding on the impact of pH on the kinetics of pH darkening on the mustard stain.

**Claims**

1. A method of measuring stain removal and/or change in colour of a fabric of multiple stains from stained fabric (1),

   wherein the stained fabric (1) comprises one or more different types of stains,
   wherein the stained fabric (1) is placed in an apparatus (2),
   wherein the apparatus (2) comprises:

   (i) a wash bath (3);
   (ii) rotating drum (4);
   (iii) compressive roller (5);
   (iv) means of applying tension to the stained fabric (6);
   (v) unstained static fabric (7);
   (vi) means of applying tension to the static fabric (8);
   (vii) image capture means (9), wherein the image capture means is a colour camera; and

(viii) image analysis means (10),

wherein the stained fabric (1) is secured under tension on the rotating drum (4)
wherein the stained fabric is wrapped around the rotaing drum and placed under stretching tension via a mechanical mechanism to avoid wrinkling/folding of the fabric during the application of abrasive force via the unstained static fabric,
wherein during each rotation, each type of stain on the stained fabric (1) is repeatedly contacted to the wash bath (3) at least once per rotation,
wherein during each rotation, each type of stain on the stained fabric (1) is contacted to the compressive roller (5) which applies a compressive force to the stain,
wherein the image capture means (9) is positioned such that the image capture means (9) measures in real time the light absorbance of each type of stain on the stained fabric (1) and a background fabric at least once during each rotation,
wherein the stained fabric (1) comprises a visual cue (11),
wherein the image capture means (9) identifies the visual cue (11), and the image analysis means (10) associates the measurement reading for a type of stain with the visual cue for that type of stain , and distinguishes between different types of stain on the stained fabric and in this manner reads multiple different types of stains or sets on the same stained fabric,
wherein the percentage of stain colour removed is calculated using the R G B components of the stains captured by the camera.

**Patentansprüche**

1. Verfahren zur Messung von Fleckenentfernung und/oder Farbveränderung eines Stoffes mit mehreren Flecken aus fleckigem Stoff (1),

wobei der fleckige Stoff (1) eine oder mehrere verschiedene Arten von Flecken umfasst,
wobei der fleckige Stoff (1) in einem Apparat (2) platziert wird,
wobei der Apparat (2) umfasst:

(i) ein Waschbad (3);
(ii) Drehtrommel (4);
(iii) Druckwalze (5);
(iv) Mittel zum Aufbringen von Spannung auf den fleckigen Stoff (6);
(v) nicht fleckiger statischer Stoff (7);
(vi) Mittel zum Aufbringen von Spannung auf den statischen Stoff (8);
(vii) Bildaufnahmemittel (9), wobei das Bildaufnahmemittel eine Farbkamera ist; und
(viii) Bildanalysemittel (10),

wobei der fleckige Stoff (1) unter Spannung auf der Drehtrommel (4) gesichert ist,
wobei der fleckige Stoff um die Drehtrommel gewickelt und unter Streckspannung über einen mechanischen Mechanismus platziert wird, um ein Knittern/Falten des Stoffes während der Aufbringung von Schleifkraft über den nicht fleckigen statischen Stoff zu vermeiden,
wobei während jeder Drehung jede Art von Flecken auf dem fleckigen Stoff (1) wiederholt mit dem Waschbad (3), mindestens einmal pro Umdrehung, in Kontakt gebracht wird,
wobei während jeder Drehung jede Art von Flecken auf dem fleckigen Stoff (1) mit der Druckwalze (5) in Kontakt gebracht wird, die eine Druckkraft auf den Flecken aufbringt,
wobei das Bildaufnahmemittel (9) so positioniert ist, dass das Bildaufnahmemittel (9) in Echtzeit die Lichtabsorption jeder Art von Flecken auf dem fleckigen Stoff (1) und mindestens einmal während jeder Umdrehung auf einem Hintergrundstoff misst,
wobei der fleckige Stoff (1) eine visuelle Hilfe (11) umfasst,
wobei das Bildaufnahmemittel (9) die visuelle Hilfe (11) erkennt und das Bildanalysemittel (10) die Messablesung für eine Art von Flecken der visuellen Hilfe dieser Art von Flecken zuordnet und zwischen verschiedenen Arten von Flecken auf dem fleckigen Stoff unterscheidet und auf diese Weise mehrere verschiedene Arten von Flecken oder Sätzen auf demselben fleckigen Stoff liest,
wobei der Prozentsatz der Fleckenfarbe unter Verwendung der R-G-B-Komponenten der von der Kamera erfassten Flecken berechnet wird.

**Revendications**

1. Procédé de mesure d'élimination des taches et/ou d'un changement de couleur d'un tissu de multiples taches d'un tissu taché (1),

   dans lequel le tissu taché (1) comprend un ou plusieurs types différents de taches,
   dans lequel le tissu taché (1) est placé dans un appareil (2),
   dans lequel l'appareil (2) comprend :

   (i) un bain de lavage (3) ;
   (ii) un tambour rotatif (4) ;
   (iii) un rouleau de compression (5) ;
   (iv) un moyen d'application de tension au tissu taché (6) ;
   (v) un tissu statique non taché (7) ;
   (vi) un moyen d'application de tension au tissu statique (8) ;
   (vii) un moyen de capture d'image (9), dans lequel le moyen de capture d'image est une caméra couleur ; et
   (viii) un moyen d'analyse d'image (10),

   dans lequel le tissu taché (1) est fixé sous tension sur le tambour rotatif (4)
   dans lequel le tissu taché est enveloppé autour du tambour rotatif et placé sous une tension d'étirement par l'intermédiaire d'un mécanisme mécanique pour éviter un froissement/pliage du tissu pendant l'application d'une force abrasive par l'intermédiaire du tissu statique non taché,
   dans lequel pendant chaque rotation, chaque type de tache sur le tissu taché (1) est mis en contact de manière répétée avec le bain de lavage (3) au moins une fois par rotation,
   dans lequel pendant chaque rotation, chaque type de tache sur le tissu taché (1) est mis en contact avec le rouleau de compression (5) qui applique une force de compression à la tache,
   dans lequel le moyen de capture d'image (9) est positionné de telle sorte que le moyen de capture d'image (9) mesure en temps réel l'absorbance de lumière de chaque type de tache sur le tissu taché (1) et un tissu d'arrière-plan au moins une fois pendant chaque rotation,
   dans lequel le tissu taché (1) comprend un signe visuel (11),
   dans lequel le moyen de capture d'image (9) identifie le signe visuel (11), et le moyen d'analyse d'image (10) associe la lecture de mesure pour un type de tache au signe visuel pour ce type de tache, et fait la distinction entre différents types de tache sur le tissu taché et de cette manière lit de multiples types différents de taches ou d'ensembles sur le même tissu taché,
   dans lequel le pourcentage de couleur de tache éliminé est calculé en utilisant les composantes R V B des taches capturées par la caméra.

Figure 1.

Figure 2.

12a   12b

Figure 3.

Figure 4.

Figure 5:

Figure 6.

Figure 7.

Figure 8.

$$SRI(\%) = \frac{|AB| - |AD|}{|AB|} \cdot 100$$

$$\left|AB\right| = \sqrt{\left(R_B - R_A\right)^2 + \left(G_B - G_A\right)^2 + \left(B_B - B_A\right)^2}$$

$$\left|AD\right| = \sqrt{\left(R_D - R_A\right)^2 + \left(G_D - G_A\right)^2 + \left(B_D - B_A\right)^2}$$

Figure 9.

Figure 10.

Figure 11.

Figure 12.

Experiment 1

Figure 13.

Experiment 2

— Experiment　— – – Prediction

Figure 14.

Figure 15.

**Experiment 4**

——— Experiment    - - - Prediction

Figure 16.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2561133 A
- CN 105628563
- GB 653657 A
- DE 102006029485

**Non-patent literature cited in the description**

- **HAILING.** *Introduction to tribology,* 1976, 61